# EUROPEAN PATENT SPECIFICATION

(11) **EP 3 927 254 B1**
(45) Date of publication and mention of the grant of the patent: **27.03.2024**
(21) Application number: 20751399.5
(22) Date of filing: 07.07.2020
(51) Int. Cl.: A61B 17/29, A61B 17/128, A61B 17/00, A61B 1/00

(54) **TISSUE CLIPPING DEVICE**
GEWEBECLIPVORRICHTUNG
DISPOSITIF DE PINCEMENT DE TISSU

(30) Priority: 29.07.2019 US 201962879874 P
(43) Date of publication of application: 29.12.2021
(73) Proprietor: Boston Scientific Scimed, Inc., Maple Grove, MN 55311 (US)
(72) Inventor: SAENZ VILLALOBOS, Gonzalo Jose, Heredia, 40101 (CR); RYAN, Shawn, Littleton, Massachusetts 01460 (US); SOLANO MONTENEGRO, Esteban, Propark, El Coyol Alajuela (CR)
(74) Representative: Vossius & Partner Patentanwälte Rechtsanwälte mbB
(86) International application number: PCT/US2020/041072
(87) International publication number: WO 2021/021397

(56) References cited:
- WO-A1-2008/070556
- GB-A- 2 151 142
- US-A- 5 820 546
- US-A1- 2002 111 534
- US-A1- 2003 135 204
- US-A1- 2009 171 159
- US-A1- 2015 087 994
- US-A1- 2018 140 300

## Description

### Priority Claim

The present disclosure claims priority to U.S. Provisional Patent Application Serial No. 62/879,874 filed July 29, 2019.

### Field

The present disclosure relates generally to devices, systems and methods for treating tissue within a naturally occurring body lumen accessed via an insertion device.

### Background

Minimally invasive procedures deep within the alimentary canal, although more and more prevalent, have also highlighted potential problems not fully addressed previously. For example, in procedures such as ECRP (Endoscopic Retrograde Cholangio-Pancreatography), the properties of currently available clips are often problematic as these and similar procedures are often best served by insertion instruments such as duodenoscopes specifically configured to observe and treat anatomical structures extending transverse to a longitudinal axis of the device. That is, for many procedures, physicians may prefer duodenoscopes including optics with viewing fields transverse to the axis along which the device extends and including ports for the insertion of treatment devices along similarly transverse axes. However, many treatment devices (e.g., hemostatic clips) are best suited for application along the longitudinal axis of the insertion device and can increase the difficulties and/or risks associated with procedures in which they are applied via transverse approaches.

US 5 820 546 A discloses an endoscope including a guiding device for a biopsy forceps instrument. The guiding device has a guiding tube that is curved or bent, wherein the bent portion can be straightened if an external force is applied.

US2002/111534 A1 discloses an endoscope having an optical system capable of viewing in the side direction. A channel, which opens on the side, has an elevator near a distal exit hole for changing the direction of a tool, such as the holding device, as it exits the distal exit hole.

### Summary

The system according to the invention is defined in claim 1. Embodiments are defined in the dependent claims. The methods disclosed are not part of the claimed invention.

### Brief Description

Fig. 1A shows a clip assembly according to a first embodiment;
Fig. 1B shows a close up partially cross-sectional view of the clip of Fig. 1;
Fig. 2 shows the clip assembly of Fig. 1 deployed from a duodenoscope;
Fig. 3 shows the clip assembly of Fig. 1 deployed with its position adjusted using an elevator of the duodenoscope;
Fig. 4 shows a cross-sectional view of a clip assembly according to a further embodiment;
Fig. 5 shows an embodiment of a coupling between a clip and a flexible insertion section;
Fig. 6 shows a second embodiment of a coupling between a clip and a flexible insertion section;
Fig. 7A shows a clip assembly including the coupling of Fig. 6 in a first configuration; and
Fig. 7B shows the clip assembly of Fig. 7A in a second configuration.
Fig. 8 shows a clip assembly according to a further embodiment.

### Detailed Description

The present disclosure may be further understood with reference to the following description and the appended drawings, wherein like elements are referred to with the same reference numerals. The present disclosure is directed to a device and method for treating tissue transverse to a longitudinal axis of an insertion instrument such as a duodenoscope. More specifically, the present embodiments are directed to devices for clipping tissue in locations offset laterally with respect to a longitudinal axis of an insertion device. It is noted that the terms proximal and distal, as used herein, refer to a direction toward (proximal) and away from (distal) a user of the device.

Although the embodiments described herein a specifically configured for the treatment of the gastrointestinal tract via a duodenoscope, those skilled in the art will understand that the embodiments described herein may also be employed to close papilla (rounded openings of internal biliary ducts) and to treat tissue defects in hard to reach locations whether approached via a laterally oriented port in a duodenoscope or through a longitudinal port in a standard endoscope. For example, duodenoscope are often used for resections in sections of the stomach for which closure could be needed. Additionally, embodiments wherein the bend is not as sharp as 90 degrees, e.g., wherein the bend is 45 degrees, can be used for front viewing scopes in scenarios where there is an awkward positioning of a defect and the standard endoscope could not be utilized.

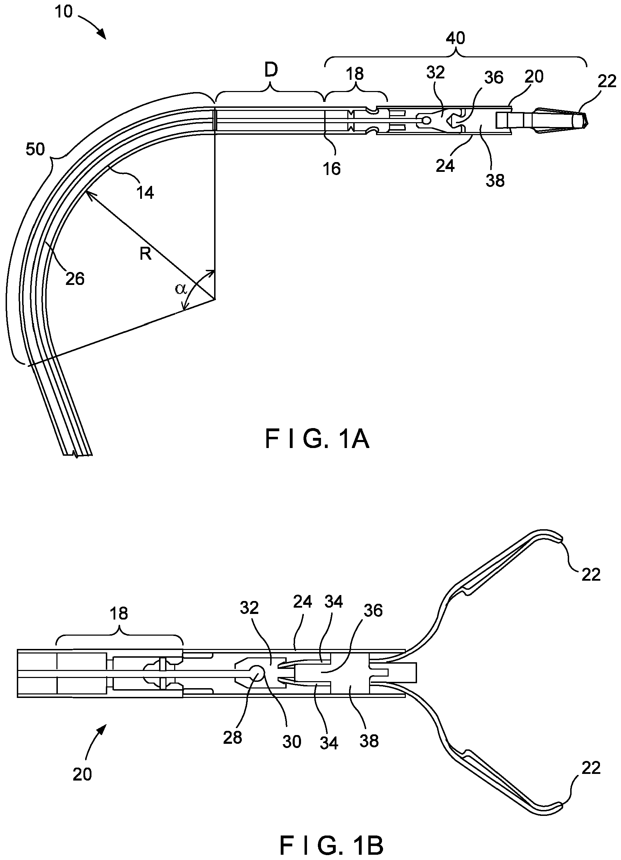

As shown in Figs. 1 - 3, a treatment device 10 according to an embodiment of the invention which in this example is a clipping device inserted through a duodenoscope 12. The device 10 includes a flexible insertion section 14 formed, in this embodiment, as a tubular coil extending from a proximal end (not shown) to a distal end 16 which is coupled to a bushing 18. The bushing 18 is releasably coupled to a clip 20 that includes a pair of clip arms 22 slidably received within a capsule 24. A control wire 26 extends from a proximal actuator (not shown) which, during use, remains accessible to a user outside the body outside the proximal end of the duodenoscope. The control wire 26, through the bushing 18 into the capsule 24 where it is coupled to the clip 20 so that movement of the control wire 26 proximally and distally through the insertion section 14 moves the clip arms 22 proximally and distally relative to the capsule 24.

Aside from the insertion section 14 and the control wire 26, the clip 20 of this embodiment may be constructed in any known manner. Thus, the specific construction of the internal construction of the clip 20 and the connection between the clip 20 and the bushing 18 are exemplary only. In this example, the control wire 26 includes an enlarged distal tip 28 that is received within a correspondingly shaped cavity 30 within a yoke 32 that is slidably received within the capsule 24. The yoke 32 includes opposed arms 34 that grip a proximally extending tab 36 of a tension member 38. The clip arms 22 are bent around the tension member 38 with proximal ends of the clip arms 22 coupled to the yoke 32.

As would be understood by those skilled in the art, although the insertion section 14 is flexible the distal portion 40 of the device 10 extending from the proximal end of the bushing 18 to the distal ends of the clip arms 22 is substantially rigid. The rigidity of this distal portion 40 can cause difficulties for users of known clips in applications in which the user wishes to deploy the clip through a port transverse to a longitudinal axis of the device through which the clip is inserted. That is, the rigidity and length of this distal portion 40 may make it difficult for such a clip to be bent around the bending radius required to exit a transverse port such as a port 42 of the duodenoscope 12. As the scopes like the duodenoscope 12 are also aimed transverse to the longitudinal axis L of the scope 12, a viewing area 46 of the camera 48 of the scope 12 is a generally conic volume extending radially away from the axis L. A standard clip passed out of the port 42 will generally extend distally away from the scope 12 outside the viewing area 46. As would be understood by those skilled in the art, a user wanting to redirect this clip toward a target tissue site within the viewing area 46 may use an elevator 52 of the scope to bend this clip. However, using the elevator 52 to force such a sharp bend in the clip risks damaging the clip and/or the scope 12.

The control wire 26 of the device 10 of the system according to the invention is pretreated to include a bend 50 so that, in a resting state, the insertion section 14 bends around a radius R. In this embodiment, the radius R may range from 5 to 15 mm. The bend 50 preferably ends a selected distance D proximal of the bushing 18 so that when the device 10 exits the port 42, the bend 50 will be seated in a portion of a working channel of the scope 12 adjacent to the elevator 52 of the scope 12. In this embodiment, the distance D may range from 0 to 12 mm; a shorter distance may be preferable so that tissue is closer to the scope 12. In this embodiment, the bend 50 extends around an arc angle a of approximately 90 degrees, with some 10 degrees of variation i.e., 80 degrees to 100 degrees, to aim the device 10 generally transverse to the axis L.

However, in other embodiments, the arc angle a may be at different angle configurations, e.g. at 45 degrees, to be utilized with front viewing scopes for awkward placed lesions. The bend 50 is imposed on the control wire 26 through a mechanical plastic deformation that bends the control wire 26 into a predetermined shape. The control wire 26 is made of a stiff and rigid material, so that the control wire 26 can withstand a force that straightens the control wire 26 and spring back into its predetermined shape. Similarly, the tubular coil has a predetermined shape that contains a bend imposed through thermal heat treatment of the tubular coil. The tubular coil can also spring back to its predetermined shape after it is forced into a straight shape. Thus, a straightening force may be applied to the control wire 26 and the tubular coil to straighten then, but after the straightening force is released, the control wire 26 and tubular coil return to their respective predetermined shape.

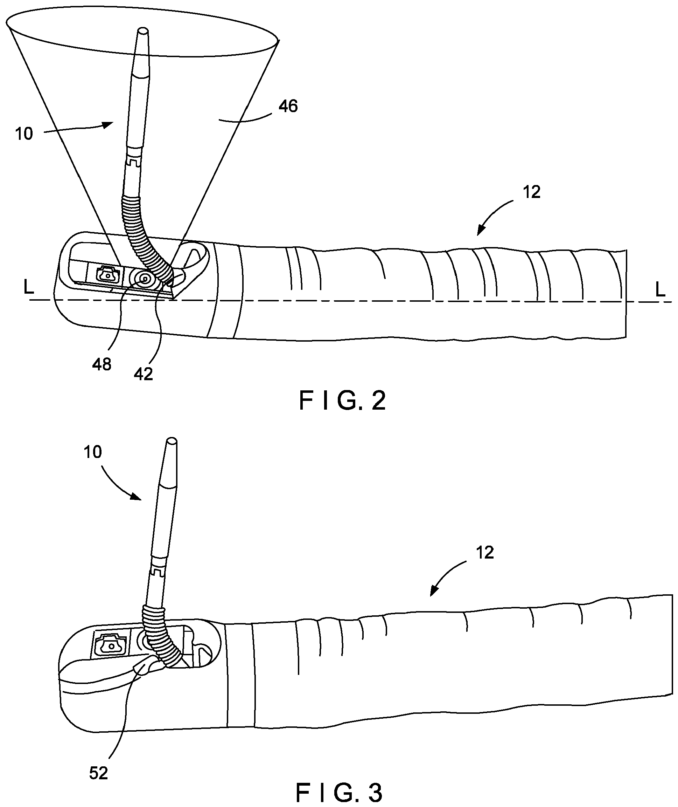

Those skilled in the art will understand that, as the device 10 according to this embodiment extends initially out of the port 42 within the viewing area 46 of the camera 48, the elevator 52 may be used as a fine correction for the aiming of the device 10 while the device 10 is visualized by the camera 48 as shown in Fig. 3. As indicated above, this arrangement of the bend 50 in the control wire 26 may be employed with any known clipping device (or other end effector) that includes a flexible insertion section to facilitate deploying the device via a transversely aimed port like the port 42 of the scope 12. The device 10 has a natural tendency to move towards a path of least resistance, thus the device 10 is capable of auto-aligning with the port 42 to exit the scope 12. In an exemplary embodiment, proximal markings may be implemented on the proximal end of the internal section 14 to allow a physician to track the device 10 as it moves out of the scope 12.

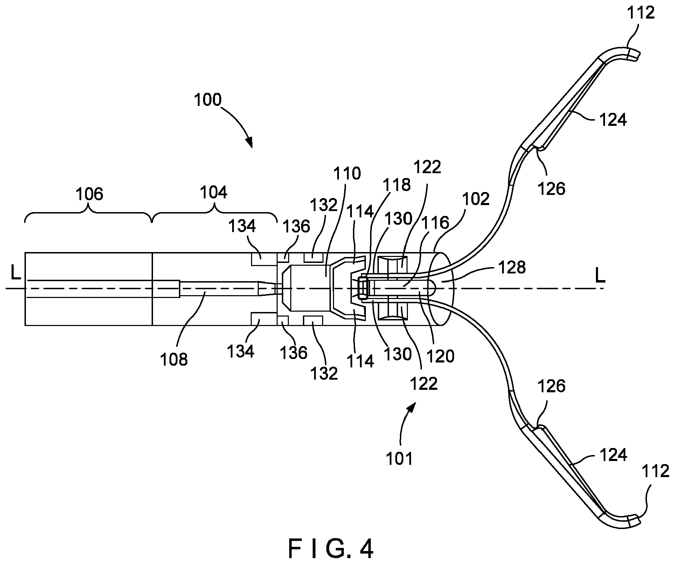

Fig. 4 shows a clipping device 100 comprising a clip 101 coupled to a bushing 104 a proximal end of which is coupled to the distal end of a flexible insertion section 106 through which a control wire 108 extends to couple to a yoke 110 that incorporates the functionality of the tension member and the yoke of the clip 20 described above into a single element. This eliminates the arms 34 of the yoke 32 that grip the tab 36 of the tension member 38 allowing a corresponding shortening of the clip mechanism which also permits the arms 114 of the yoke 110 to move further distally permitting a corresponding shortening of the clip arms.

Those skilled in the art will understand that the clipping device 100 may be employed with any standard insertion section and control wire or may include a control wire/insertion section combination that includes a bend 50 as described above for the control wire 26 and the insertion section 14. The maneuverability of the device 100 according to this embodiment is further enhanced by the structure of the internal mechanism coupling the control wire 108 to clip arms 112 that enables the rigid portion of the device 100 extending from the proximal end of the bushing 104 distally through a capsule 102 to distal ends of the clip arms 112.

The yoke 110 includes a pair of arms 114 extending from a mid-section thereof and includes a clip arms retaining section 116 coupled to a proximal portion of the yoke 110 (e.g., by a weld or integrally formed with the yoke 110) via frangible joint 118 which may be formed, for example, as narrowed or weakened section of the clip arms retaining section 116. Distally of the frangible joint 118, the clip arms retaining section 116 includes a tongue 120 received between the clip arms 112 to bias the clip arms 112 away from one another so that, when the clip arms 112 are advanced distally out of the capsule 102, the clip arms 112 spread apart into a tissue receiving configuration as shown in Fig. 4. This arrangement also allows the proximal portions of the clip arms 112 to be shorter than in the clip 20 reducing the over-all length of the clip 101. The clip arms retaining section 116 also includes opposed orienting fingers 122 each of which passes through a corresponding opening in one of the clip arms 112 to maintain a desired orientation of the clip arms 112 as they are moved proximally and distally through the capsule 102.

The orienting fingers 122, in this embodiment, are also sized so that they extend substantially across and slidingly engage opposed surfaces of the capsule 102 to ensure that the clip arms 112 move smoothly proximally and distally as the control wire 108 is advanced or retracted through the insertion section 106. The clip arms 112 include widened sections 124 and proximal abutting surfaces 126 that define a maximum extent to which the clip arms 112 can be drawn proximally into the capsule 102. That is, the abutting surfaces 126 extend laterally relative to the longitudinal axis L of the device 100 to an extent greater than a diameter of an opening 128 so that, as the clip arms 112 are drawn proximally into the capsule 102, eventually the abutting surfaces 126 contact the end of the capsule 102 surrounding the opening 128.

To deploy the clip 101 when it has been positioned gripping target tissue as desired, the user withdraws the control wire 108 proximally (e.g., by actuating an actuator on a proximal handle (not shown) until the abutting surfaces 126 contact the capsule 102. The user will feel resistance to further proximal movement of the control wire 108. The user then applies increasing tension to the control wire 108 until, when a predetermined level of tension is reached, the frangible joint 118 fails and the yoke 110 is drawn proximally through the capsule 102 away from the clip arms retaining section 116. As the yoke 110 moves proximally away from the clip arms retaining section 116, the arms 114 of the yoke move away from proximal ends 130 of the clip arms 112 freeing the proximal ends 130 of the clip arms 112 to spring outward so that locking features on the proximal ends 130 of the clip arms 112 spring radially outward away from the axis L to engage locking features 132 on the capsule 102. This locks the clip arms 112 in a tissue gripping configuration in which the distal ends of the clip arms 112 are drawn together toward the axis L to grip tissue positioned therebetween.

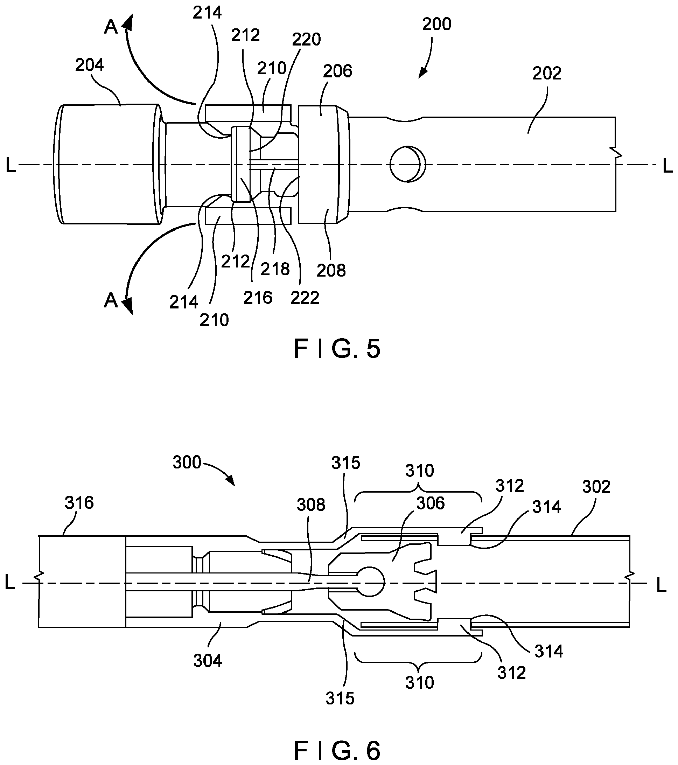

In addition, after the yoke 110 is separated from the clip arms retaining section 116 it is drawn further proximally out of the proximal end of the capsule 102. At this point the arms 114 of the yoke 110 which extend further radially away from the axis L than do more proximal portions of the yoke 110 engage bushing coupling features 134 which grip corresponding coupling features 136 of the capsule 102. As the yoke 110 is drawn proximally into the bushing 104, the arms 114 push the bushing coupling features 134 radially outward away from the axis L moving them out of engagement with the coupling features 136 of the capsule 102 and separating the clip 101 from the bushing 104 and the insertion section 106 and leaving the clip 101 locked on the gripped portion of target tissue while the rest of the device 100 is withdrawn from the body. As would be understood by those skilled in the art, any known coupling by which a bushing is releasably coupled to the capsule of a clip can be employed including the coupling of Fig. 5 described below.

As shown in Fig. 5, a coupling 200 between a capsule 202 of a clipping device (e.g., a device 100) and a bushing 204 includes a coupler 206. A distal end of the coupler 206 is formed as a tube 208 coupled to the capsule 202 while a proximal end of the coupler 206 includes a plurality of arms 210 distributed around a circumference of the tube 208. Each of the arms 210 includes a recess 212 having a proximal locking surface 214 that grips a radial projection 216 of the bushing 204. In this embodiment, the radial projection 216 is formed as an annular surface projecting radially outward away from the axis L to extend generally perpendicularly away from an outer surface of the bushing 204. The proximal locking surfaces 214 of the arms 210 of this embodiment are also generally perpendicular to the axis L so that the capsule 202 is firmly coupled to the bushing 204 via the coupler 206.

A distal surface of each of the recesses 212 is formed as a ramp 218 extending distally away from a radially outer edge of distal surface 220 of the radial projection 216 at an angle (e.g., the angle ranging between 30 and 45 degrees from an interior surface of the arm 210) so that, a radially outermost end of each of the ramps 218 contacts the distal surface 220 while a gap is formed between more radially inwardly located portions of the ramps 218 and the distal surface 220. Thus, as a clip including the coupling 200 is inserted through an insertion device to a target site within the body, the arms 210 securely lock the capsule 202 to the bushing 204. However, when, as described above, a clip is drawn into the capsule to a point at which it can be moved no further proximally into the capsule 202, the user applies increasing proximally directed force to a control member 222 which is opposed by a distally directed force as the insertion section 106 resists compression.

As this compression is applied to the coupler 206, the ramps 218 slide proximally against the distal surface 220 of the bushing 204 causing the proximal ends of the arms 210 to rotate outward in the direction of the arrows A of Fig. 5. This moves the proximal locking surfaces 214 of the arms 210 out of engagement with the radial projection 216 until the radial projection 216 is disengaged from the recesses 212, thereby separating the bushing 204 from the coupler 206. Therefore, the tube 208 and the capsule 202 may rotate freely about axis L. In another embodiment, a triple wire configuration may be used to produce a control wire driven rotation, wherein a user rotates the clip so that it can be placed as needed. In another embodiment, the capsule 202 may be fixed to the bushing 204, thus, no rotation about axis L may occur.

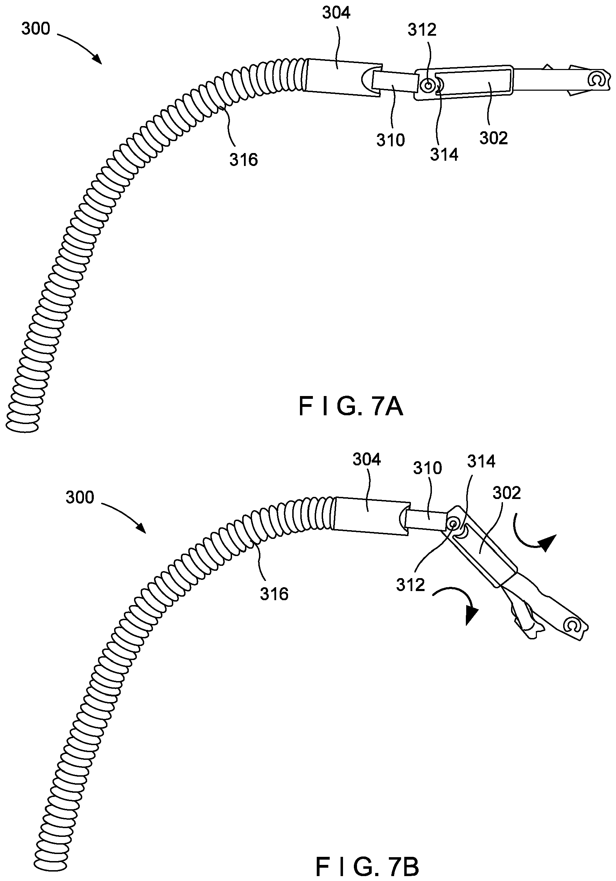

As shown in Figs. 6, 7A and 7B, a device 300 including a coupling mechanism according to a second embodiment includes a capsule 302 coupled to a bushing 304 and housing a yoke 306 to which a control wire 308 is attached. The yoke 306 operate in conjunction with a tension member (not shown) or any other known clipping mechanism so long as, when the clip is deployed, the yoke 306 is freed to move proximally with the control wire 308 out of the capsule 302 into the bushing 304. As will be seen below the bushing includes opposed arms 310 that extend around a proximal portion of the capsule 302 so that diametrically opposed pins 312 at the distal end of the bushing 304 extend into receptacles 314 formed in the wall of the capsule 302. The arms 310 are flared outward via ramped portions 315. As the arms 310 extend around the outside of the capsule 302, the arms 310 are separated from one another by a distance greater than a diameter of the capsule 302 while the ramped portions 315 define a reduced diameter portion of the bushing 304 having a diameter less than that of the capsule 302 so that, as described below in more detail, radially outer edges of the yoke 306 which is slidable within the capsule 302 will impact and push radially outward the ramped portions 315 as the yoke 306 is drawn proximally through the bushing 304.

However, those skilled in the art will understand that the outer diameter of the bushing 304 may remain consistent throughout its length so long as the inner diameter decreases (in at least one direction) so that the radially outer surfaces of the yoke 306 contact the inner surface of at least a portion of the bushing 304 to drive the arms 310 radially outward after the clip has been deployed and the yoke 306 is drawn proximally through the bushing 304. Thus, as the yoke 306 contacts the ramped portions 315 and drives them radially outward the pins 312 are pushed radially outward out of the receptacles 314, freeing the capsule 302 from the bushing 304 and separating the clip from the insertion section 316, the bushing 304, the control wire 308 and the entire proximal portion of the device.

Furthermore, as would be understood by those skilled in the art, if the receptacles 314 and the pins 312 are made circular, the capsule 302 will be rotatable relative to the bushing 304 and the insertion section 316. This rotation will, of course, be limited by any resistance to bending of the control wire 308. However, this rotation (as shown in Figs. 7A and 7B) can further aid in facilitating the application of a clip through a path including tight bends such as, for example, as may be encountered when a clip is passed longitudinally through a duodenoscope and then must bend through an arc of approximately 90 degrees to exit the scope via a laterally facing port.

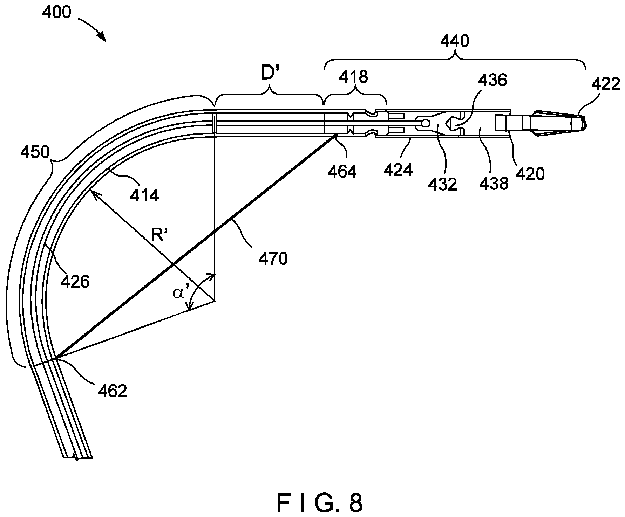

As shown in Fig. 8, a treatment device 400 may be substantially similar to the treatment device 10 described above. The device 400 may include a control wire 426, an insertion section 414, a bushing 418, a clip 420 containing clip arms 422, and a distal portion 440. Similar to the distal portion 40 described above, the distal portion 440 extends from a proximal end of the bushing 418 to distal ends of the clip arms 422. The distal portion 440 includes a capsule 424. The capsule 424 houses a coupling mechanism similar to that of devices 10 and 100-300. The coupling mechanism includes a yoke 432, a tab 436, a tension member 438, and the clip 420. Moreover, the control wire 426 has a bend 450, similar to the bend 50 of the control wire 26, extending from a proximal end (not shown) to a distal end having a radius R' similar to the radius R of the device 10. In this embodiment, the bend 450 extends around an arc angle a', the angle a' being similar to the angle α of the device 10. In this embodiment, a distance D' is between the distal end of the bend 450 and a proximal end of the bushing 418, the distance D' being similar to the distance D of the device 10.

The device 400 includes a first hole 462 at the proximal end of the bend 450 and a second hole 464 at the proximal end of the distal portion 440. The first hole 462 and the second hole 464 are sized and shaped so that an actuation cord 470 can extend therebetween to allow for better location of the clip 420. The cord 470 extends from a proximal end (not shown) to a distal end, at the proximal end of the distal portion 440. A portion of the cord 470 extends through the insertion section 414 to the first hole 462. The proximal end of the cord 470 is accessible to a user, such that the user may pull proximally on the proximal end of the cord 470 to straighten the cord 470, thus forming a hypothenuse between the first hole 462 and the second hole 464 and further curving the bend 450. By applying different amounts of force when pulling the proximal end of the cord 470, the user controls bending of the bend 450 and a location of the distal portion 440.

As would be further understood, a clip assembly combining such a pivoting connection between the capsule and the bushing with a control wire (e.g., the control wire 26 described above) having a bend adjacent the distal end of the insertion section, may show significant improvement in operations with lateral ports and/or in procedures requiring access to hard to reach locations requiring tight bending radii. Such a combination may be further improved by employing the shorter clip mechanism of the device 100 described above. Such a device would be effective in either an insertion device such as an endoscope with a longitudinally oriented exit port to its working channel or with the lateral portion of the duodenoscopes described above to, for example, close lesions in complicated positions in the large intestine or other portions of the gastroenterological tract, in confined spaces or where the view available to the scope is oblique.

Those skilled in the art will understand that there are various modifications that may be made to the embodiments described without departing from the teachings of this application. For example, although the embodiments have been described in regard to the treatment of internal bleedings and closing resection defects or holes in tissue, those skilled in the art will understand that the embodiments may be modified to be used for similar structural applications. For the similar structural applications, an insertion section can be included for biopsy forceps, wherein a curvature of the insertion section may be used for obtaining samples in awkward or oblique locations. Additionally, the embodiments may be modified to include 30-, 45-, or 60-degree bends to suit different user needs and to be utilized on front viewing scopes.

## Claims

1. A system for treating tissues, comprising:
an insertion device, the insertion device including a port, a camera, and a working channel extending therethrough along a longitudinal axis; and
a tissue treating device (10, 400) comprising:
a control wire/insertion section combination including
a flexible insertion section (14, 414) extending from a distal end (16) which, during use is inserted to a target site within a living body to a proximal end which, during use remains outside the body; and
a control wire (26, 108, 308, 426) received within the insertion section (14, 414) and extending from a proximal end coupled to an actuator, the control wire/insertion section combination including a bend (50, 450) in a distal portion thereof configured so that, in a resting state, the control wire (26, 108, 308, 426) and the insertion section (14, 414) bend through a predetermined arc at a selected bending radius, a distal portion of the control wire (26, 108, 308, 426) being configured to pass into and through the working channel of the insertion device without plastic deformation of the control wire (26, 108, 308, 426);
an end effector coupled to a distal end of the insertion section (14, 414); and
**characterised in that** the system comprises:
an elevator (52) at a distal end of the insertion device that changes the bend (50, 450) of the control wire/insertion section combination.

2. The system of claim 1, wherein the insertion section (14, 414) includes a bushing (18, 418) a distal end of which is coupled to the end effector, the end effector being a tissue clipping device (100).

3. The system of claim 2, wherein the tissue clipping device includes a slidable element (32, 110, 306) coupled to a distal end of the control wire (26, 108, 308, 426), the slidable element (32,110,306) being coupled to tissue gripping arms of the tissue clipping device so that, as the control wire (26, 108, 308, 426) is moved proximally and distally within the insertion section (14, 414), the slidable element (32,110,306) slides proximally and distally within a capsule of the tissue clipping device to move the arms into and out of the capsule.

4. The system of claim 3, wherein a proximal portion of the slidable element (32,110,306) includes an abutting surface at a radially outer portion thereof sized to engage, as the proximal portion of the slidable element (32,110,306) is drawn proximally out of the capsule into the bushing (18, 418), the radially outer portion of the slidable element (32,110,306) pushes a portion of the bushing (18, 418) radially outward to disengage a locking structure of the bushing (18, 418) from a corresponding locking structure of the capsule to separate the capsule from the bushing (18, 418).

5. The system of any of claims 1-4, wherein a bend (50, 450) in the control wire (26, 108, 308, 426) is configured so that, in a resting state the insertion section (14, 414) bends through an arc of approximately 90 degrees.

6. The system of any of claims 1-5, wherein a bend (50, 450) in the control wire (26, 108, 308, 426) is configured so that, in a resting state the insertion section (14, 414) bends through an arc of approximately 45 degrees.

7. The system of claim 3, wherein a distal portion of the slidable element (32,110,306) further comprises
opposed orienting fingers each of which sized and shaped to pass through a corresponding opening in one of the arms to maintain a desired orientation of the arms.

8. The system of claim 3, further comprising:
widened sections and proximal abutting surfaces on the arms that define a maximum extent to which the arms can be drawn proximally into the capsule.

9. The system of claim 2, wherein the bushing (18, 418) is coupled to a capsule of the tissue clipping device via a coupler, the coupler including a plurality of arms distributed around a circumference of the coupler, each of the arms including a recess sized and shaped to engage a locking wall of the bushing (18, 418) to couple the bushing (18, 418) to the coupler, each of the recesses including a proximal surface and a distal surface engaging the locking wall of the bushing (18, 418), a first one of the proximal and distal surfaces forming a ramp angled relative to a surface of the locking wall with which the ramp is in contact, the ramps being oriented so that, when a predetermined compression is applied between the bushing (18, 418) and the coupler, the ramps slide over and disengage from the locking wall to decouple toe coupler and the capsule from the bushing (18, 418).

10. The system of claim 4, wherein the bushing (18, 418) includes first and second arms that extend distally over a portion of the capsule, each of the arms including a pin that extends into a corresponding receptacle formed in an outer wall of the capsule, the pins forming a pivoting connection permitting rotation of the capsule relative to the bushing (18, 418).

11. The system of claim 3, wherein the slidable element (32,110,306) includes a yoke and a tension member coupled to one another via a frangible link.

12. The system of any of claims 1-11, further comprising:
a first hole at a proximal end of the bend (50, 450);
a second hole at a proximal end of the end effector; and
an actuation cord, extending from a proximal end coupled to an actuator that remains accessible to a user during use to a distal end coupled to the end effector, wherein the cord passes through the insertion section (14, 414), exits the first hole, enters the second hole, and couples to a portion of the device distal of the bend (50, 450).

13. The system of any of claims 1-12, wherein the camera of the insertion device is aimed transverse to the longitudinal axis of the insertion device, producing a viewing area in a generally conic volume extending radially away from the axis.

## Patentansprüche

1. System zur Behandlung von Geweben, das aufweist:
eine Einführungsvorrichtung, wobei die Einführungsvorrichtung eine Öffnung, eine Kamera und einen Arbeitskanal aufweist, der sich entlang einer Längsachse durch sie hindurch erstreckt; und
eine Gewebebehandlungsvorrichtung (10, 400), die aufweist:
eine Steuerdraht/Einführungsabschnitt-Kombination, die aufweist:
einen flexiblen Einführungsabschnitt (14, 414), der sich von einem distalen Ende (16), das während der Verwendung in eine Zielstelle innerhalb eines lebenden Körpers eingeführt wird, zu einem proximalen Ende erstreckt, das während der Verwendung außerhalb des Körpers verbleibt; und
einen Steuerdraht (26, 108, 308, 426), der im Einführungsabschnitt (14, 414) aufgenommen ist und sich von einem proximalen Ende aus erstreckt, das mit einem Aktuator gekoppelt ist, wobei die Steuerdraht/Einführungsabschnitt-Kombination eine Biegung (50, 450) in einem distalen Abschnitt davon aufweist, die so konfiguriert ist, dass in einem Ruhezustand der Steuerdraht (26, 108, 308, 426) und der Einführungsabschnitt (14, 414) durch einen vorbestimmten Bogen mit einem ausgewählten Biegeradius gebogen sind, wobei ein distaler Abschnitt des Steuerdrahtes (26, 108, 308, 426) konfiguriert ist, in und durch den Arbeitskanal der Einführungsvorrichtung ohne plastische Verformung des Steuerdrahtes (26, 108, 308, 426) zu verlaufen;
einen Endeffektor, der mit einem distalen Ende des Einführungsabschnitts (14, 414) gekoppelt ist; und
**dadurch gekennzeichnet, dass** das System aufweist:
eine Heber (52) an einem distalen Ende der Einführungsvorrichtung, die die Biegung (50, 450) der Steuerdraht/Einführungsabschnitt-Kombination verändert.

2. System nach Anspruch 1, wobei der Einführungsabschnitt (14, 414) eine Hülse (18, 418) umfasst, deren distales Ende mit dem Endeffektor gekoppelt ist, wobei der Endeffektor eine Gewebeklammervorrichtung (100) ist.

3. System nach Anspruch 2, wobei die Gewebeklammervorrichtung ein verschiebbares Element (32, 110, 306) umfasst, das mit einem distalen Ende des Steuerdrahtes (26, 108, 308, 426) gekoppelt ist, wobei das verschiebbare Element (32, 110, 306) mit Gewebegreifarmen der Gewebeklammervorrichtung gekoppelt ist, so dass, wenn der Steuerdraht (26, 108, 308, 426) proximal und distal innerhalb des Einführungsabschnitts (14, 414) bewegt wird, das verschiebbare Element (32, 110, 306) proximal und distal innerhalb einer Kapsel der Gewebeklammervorrichtung gleitet, um die Arme in die und aus der Kapsel zu bewegen.

4. System nach Anspruch 3, wobei ein proximaler Abschnitt des verschiebbaren Elements (32, 110, 306) eine Stoßfläche an einem radial äußeren Abschnitt desselben aufweist, die so bemessen ist, dass sie in Eingriff kommt, wenn der proximale Abschnitt des verschiebbaren Elements (32, 110, 306) proximal aus der Kapsel in die Hülse (18, 418) gezogen wird, drückt der radial äußere Teil des gleitfähigen Elements (32, 110, 306) einen Teil der Hülse (18, 418) radial nach außen, um eine Verriegelungsstruktur der Hülse (18, 418) von einer entsprechenden Verriegelungsstruktur der Kapsel zu lösen, um die Kapsel von der Hülse (18, 418) zu trennen.

5. System nach einem der Ansprüche 1 bis 4, wobei eine Biegung (50, 450) im Steuerdraht (26, 108, 308, 426) so konfiguriert ist, dass sich der Einführungsabschnitt (14, 414) in einem Ruhezustand durch einen Bogen von ungefähr 90 Grad biegt.

6. System nach einem der Ansprüche 1 bis 5, wobei eine Biegung (50, 450) im Steuerdraht (26, 108, 308, 426) so konfiguriert ist, dass sich der Einführungsabschnitt (14, 414) in einem Ruhezustand durch einen Bogen von ungefähr 45 Grad biegt.

7. System nach Anspruch 3, wobei ein distaler Abschnitt des verschiebbaren Elements (32, 110, 306) ferner aufweist:
gegenüberliegende Ausrichtungsfinger, von denen jeder bemessen und geformt ist, durch eine entsprechende Öffnung in einem der Arme zu gehen, um eine gewünschte Ausrichtung der Arme beizubehalten.

8. System nach Anspruch 3, das ferner aufweist:
verbreiterte Abschnitte und proximale Auflageflächen an den Armen, die ein maximales Ausmaß definieren, bis zu dem die Arme proximal in die Kapsel gezogen werden können.

9. System nach Anspruch 2, wobei die Hülse (18, 418) mit einer Kapsel der Gewebeklammervorrichtung über einen Koppler gekoppelt ist, wobei der Koppler mehrere Arme aufweist, die um einen Umfang des Kopplers verteilt sind, wobei jeder der Arme eine Aussparung aufweist, die bemessen und geformt ist, in eine Verriegelungswand der Hülse (18, 418) einzugreifen, um die Hülse (18, 418) mit dem Koppler zu koppeln, wobei jede der Aussparungen eine proximale Oberfläche und eine distale Oberfläche aufweist, die in die Verriegelungswand der Hülse (18, 418) eingreift, wobei eine erste der proximalen und distalen Oberflächen eine Rampe bildet, die relativ zu einer Oberfläche der Verriegelungswand angewinkelt ist, mit der die Rampe in Kontakt ist, wobei die Rampen so ausgerichtet sind, dass, wenn ein vorbestimmter Druck zwischen der Hülse (18, 418) und dem Koppler ausgeübt wird, die Rampen über die Verriegelungswand gleiten und sich von ihr lösen, um den Koppler und die Kapsel von der Hülse (18, 418) zu entkoppeln.

10. System nach Anspruch 4, wobei die Hülse (18, 418) einen ersten und einen zweiten Arm aufweist, die sich distal über einen Teil der Kapsel erstrecken, wobei jeder der Arme einen Stift aufweist, der sich in eine entsprechende Aufnahme erstreckt, die in einer Außenwand der Kapsel ausgebildet ist, wobei die Stifte eine Schwenkverbindung bilden, die eine Drehung der Kapsel relativ zur Hülse (18, 418) ermöglicht.

11. System nach Anspruch 3, wobei das verschiebbare Element (32, 110, 306) ein Joch und ein Zugelement umfasst, die über eine zerbrechliche Verbindung miteinander gekoppelt sind.

12. System nach einem der Ansprüche 1 bis 11, das ferner aufweist:
ein erstes Loch an einem proximalen Ende des Bogens (50, 450);
ein zweites Loch an einem proximalen Ende des Endeffektors; und
eine Betätigungsschnur, die sich von einem proximalen Ende, das mit einem Aktuator gekoppelt ist, der während der Benutzung für einen Benutzer zugänglich bleibt, zu einem distalen Ende erstreckt, das mit dem Endeffektor gekoppelt ist, wobei die Schnur durch den Einführungsabschnitt (14, 414) verläuft, aus dem ersten Loch austritt, in das zweite Loch eintritt und mit einem Teil der Vorrichtung distal der Biegung (50, 450) gekoppelt ist.

13. System nach einem der Ansprüche 1 bis 12, wobei die Kamera der Einführungsvorrichtung quer zur Längsachse der Einführungsvorrichtung ausgerichtet ist und einen Sichtbereich in einem allgemein konischen Volumen erzeugt, das sich radial von der Achse weg erstreckt.

## Revendications

1. Système pour traiter des tissus, comprenant :
un dispositif d'insertion, le dispositif d'insertion comprenant un orifice, une caméra et un canal de travail s'étendant à travers ce dernier le long d'un axe longitudinal ; et
un dispositif de traitement de tissu (10, 400) comprenant :
une combinaison de fil de commande/section d'insertion comprenant :
une section d'insertion flexible (14, 414) s'étendant à partir d'une extrémité distale (16) qui, pendant l'utilisation, est insérée dans un site cible à l'intérieur d'un corps vivant jusqu'à une extrémité proximale qui, pendant l'utilisation, reste à l'extérieur du corps ; et
un fil de commande (26, 108, 308, 426) reçu à l'intérieur de la section d'insertion (14, 414) et s'étendant à partir d'une extrémité proximale couplée à un actionneur, la combinaison de fil de commande/section d'insertion comprenant un coude (50, 450) dans sa partie distale, configurée de sorte que, dans un état de repos, le fil de commande (26, 108, 308, 426) et la section d'insertion (14, 414) se plient sur un arc prédéterminé à un rayon de courbure sélectionné, une partie distale du fil de commande (26, 108, 308, 426) étant configurée pour passer dans et à travers le canal de travail du dispositif d'insertion sans déformation plastique du fil de commande (26, 108, 308, 426) ;
un effecteur terminal couplé à une extrémité distale de la section d'insertion (14, 414) ; et
**caractérisé en ce que** le système comprend :
un élévateur (52) au niveau d'une extrémité distale du dispositif d'insertion qui modifie le coude (50, 450) de la combinaison de fil de commande/section d'insertion.

2. Système selon la revendication 1, dans lequel la section d'insertion (14, 414) comprend une douille (18, 418), dont une extrémité distale est couplée à l'effecteur terminal, l'effecteur terminal étant un dispositif de pincement de tissu (100).

3. Système selon la revendication 2, dans lequel le dispositif de pincement de tissu comprend un élément coulissant (32, 110, 306) couplé à une extrémité distale du fil de commande (26, 108, 308, 426), l'élément coulissant (32, 110, 306) étant couplé aux bras de préhension de tissu du dispositif de pincement de tissu de sorte que, lorsque le fil de commande (26, 108, 308, 426) est déplacé de manière proximale et distale à l'intérieur de la section d'insertion (14, 414), l'élément coulissant (32, 110, 306) coulisse de manière proximale et distale à l'intérieur d'une capsule du dispositif de pincement de tissu pour déplacer les bras dans et hors de la capsule.

4. Système selon la revendication 3, dans lequel une partie proximale de l'élément coulissant (32, 110, 306) comprend une surface de butée au niveau de sa partie radialement externe dimensionnée pour mettre en prise, lorsque la partie proximale de l'élément coulissant (32, 110, 306) est tirée de manière proximale hors de la capsule dans la douille (18, 418), la partie radialement externe de l'élément coulissant (32, 110, 306) pousse une partie de la douille (18, 418) radialement vers l'extérieur pour dégager une structure de verrouillage de la douille (18, 418) d'une structure de verrouillage correspondante de la capsule afin de séparer la capsule de la douille (18, 418).

5. Système selon l'une quelconque des revendications 1 à 4, dans lequel un coude (50, 450) dans le fil de commande (26, 108, 308, 426) est configuré de sorte que, dans un état de repos, la section d'insertion (14, 414) se courbe sur un arc d'approximativement 90 degrés.

6. Système selon l'une quelconque des revendications 1 à 5, dans lequel un coude (50, 450) dans le fil de commande (26, 108, 308, 426) est configuré de sorte que, dans un état de repos, la section d'insertion (14, 414) se courbe sur un arc d'approximativement 45 degrés.

7. Système selon la revendication 3, dans lequel une partie distale de l'élément coulissant (32, 110, 306) comprend en outre :
des doigts d'orientation opposés, dont chacun est dimensionné et formé pour passer à travers une ouverture correspondante dans l'un des bras afin de maintenir une orientation souhaitée des bras.

8. Système selon la revendication 3, comprenant en outre :
des sections élargies et des surfaces de butée proximales sur les bras qui définissent une étendue maximum sur laquelle les bras peuvent être tirés de manière proximale dans la capsule.

9. Système selon la revendication 2, dans lequel la douille (18, 418) est couplée à une capsule du dispositif de pincement de tissu via un coupleur, le coupleur comprenant une pluralité de bras répartis autour d'une circonférence du coupleur, chacun des bras comprenant un évidement dimensionné et formé pour mettre en prise une paroi de verrouillage de la douille (18, 418) afin de coupler la douille (18, 418) au coupleur, chacun des évidements comprenant une surface proximale et une surface distale mettant en prise la paroi de verrouillage de la douille (18, 418), une première des surfaces proximale et distale formant une rampe coudée par rapport à une surface de la paroi de verrouillage avec laquelle la rampe est en contact, les rampes étant orientées de sorte que, lorsqu'une compression prédéterminée est appliquée entre la douille (18, 418) et le coupleur, les rampes coulissent sur et de dégagent de la paroi de verrouillage afin de découpler le coupleur et la capsule de la douille (18, 418).

10. Système selon la revendication 4, dans lequel la douille (18, 418) comprend des premier et deuxième bras qui s'étendent de manière distale sur une partie de la capsule, chacun des bras comprenant une broche qui s'étend dans un réceptacle correspondant formé dans une paroi externe de la capsule, les broches formant un raccordement pivotant permettant la rotation de la capsule par rapport à la douille (18, 418).

11. Système selon la revendication 3, dans lequel l'élément coulissant (32, 110, 306) comprend une culasse et un élément de tension couplé entre eux via une liaison cassable.

12. Système selon l'une quelconque des revendications 1 à 11, comprenant en outre :
un premier trou au niveau d'une extrémité proximale du coude (50, 450) ;
un deuxième trou au niveau d'une extrémité proximale de l'effecteur terminal ; et
un cordon d'actionnement, s'étendant à partir d'une extrémité proximale couplée à un actionneur qui reste accessible à un utilisateur pendant l'utilisation, à une extrémité distale couplée à l'effecteur terminal, dans lequel le cordon passe par la section d'insertion (14, 414), sort du premier trou, entre dans le deuxième trou et se couple à une partie du dispositif à distance du coude (50, 450).

13. Système selon l'une quelconque des revendications 1 à 12, dans lequel la caméra du dispositif d'insertion est orientée de manière transversale par rapport à l'axe longitudinal du dispositif d'insertion, produisant une zone de vision dans un volume généralement conique s'étendant radialement à l'opposé de l'axe.
